# EUROPEAN PATENT SPECIFICATION

(11) **EP 2 277 508 B1**
(45) Date of publication and mention of the grant of the patent: **25.04.2012**
(21) Application number: 09165106.7
(22) Date of filing: 09.07.2009
(51) Int. Cl.: A61K 9/127, A61K 9/133, A61K 48/00

(54) **Emulation of lipoprotein structures**
Nachahmung von Lipoproteinstrukturen
Émulation de structures de lipoprotéine

(43) Date of publication of application: 26.01.2011
(73) Proprietor: Marina Biotech, Inc., Bothell WA 98021-7266 (US)
(72) Inventor: Panzner, Steffen, 06114 Halle (DE); Siepi, Evgenios, 5350 Frenaros (CY)
(74) Representative: MacLean, Martin Robert

(56) References cited:
- WO-A-02/066012
- WO-A-2007/064857
- WO-A-2008/137758

## Description

### Field of the invention

The present invention relates to liposomes that overcome a lipoprotein mediated uptake blockade. More specifically, this invention relates to improvements in or relating to liposomes wherein a portion of their polar lipid head groups comprise guanido moieties or derivatives thereof.

### Background to the invention

Liposomes have widespread use as carriers for active ingredients. Neutral or negatively charged liposomes are often sued for the delivery of small molecule drugs, whereas positively charged (cationic) or the recently introduced class of amphoteric liposomes are mainly used for the delivery of nucleic acids that is plasmids or oligonucleotides. Important examples for cationic liposomes used for the delivery of nucleic acid cargoes include, but are not limited to Akinc et al., nat biotech (2008) 26:561 -569; Chien et al., Cancer Gene Ther (2005) 12:321 - 328; de Fougerolles, Nat Rev Drug Discov (2007) 6:443 - 453; Kim et al., Mol Ther (2006) 14:343 - 350; Morrissey, nat biotech (2005) 23: 1002 - 1007; Peer, Science (2008) 319: 627 - 630 or Santel, Gene Ther (2006) 13: 1222 - 1234.

WO 02/066012 discloses the general concept of amphoteric liposomes having a stable phase at both low and neutral pHs. WO 02/066012 and also WO07/107304 describe a method of loading such particles with nucleic acids starting from a low pH. Application of these liposomes for the delivery of nucleic acids has been demonstrated in Andreakos, Arthritis Rheum (2009) 60:994 - 1005. Furthermore, WO 08/043575 reveals strategies for the optimization of stability, fusogenicity and cellular transfection of amphoteric liposomes.

The amphoteric liposomes according to the abovementioned references are potent transfectants of cells. It was however observed, that the function of some of these liposomes could be blocked by the addition of certain sera, thereby potentially limiting the activity of these liposomes for the targeting of certain cells in vivo. This is further illustrated in example 3.

A more detailed investigation revealed lipoproteins as mediators of this inhibitory effect. As shown in example 4, a human serum being deficient of lipoproteins is no longer able to inhibit the uptake of liposomes as indicated by the functional delivery of siRNA to the challenged cells.

### Object of the invention

It was therefore an object of the invention to provide liposomes that can transfect cells in the presence of various sera.

Another object of the invention is to provide pharmaceutical compositions comprising such liposomes as a carrier for the delivery of active agents or ingredients, including drugs such as nucleic acid drugs, e.g., oligonucleotides and plasmids into cells or tissues.

### Summary of the Invention

The present invention provides lipid assemblies, liposomes and their use for transfection of cells according to claims 1, 5 and 11. Further advantageous embodiments of the invention are described in the dependent claims.

It was found that serum resistant transfection can be achieved with liposomes having an outer surface comprising a mixture of anionic and cationic moieties; wherein at least a portion of the cationic moieties are guanido groups.

### Detailed description of the invention

The lipoproteins competing with the transfection of liposomes comprise a variety of structures, according to their density these are known as chylomicrons, VLDL, LDL, IDL or HDL particles. In the endogenous pathway, chylomicrons are synthesized in the epithelial lining of the small intestine and are assembled using ApoB-48, a shorter variant of the ApoB gene product. Further exchange of lipoproteins with HDL particles leads to transfer of ApoC-II and ApoE to the chylomicron particle, the first mediating the activation of lipoprotein lipase, an enzyme needed for the release of lipids from the particle. The hydrolyzed chylomicrons form so called remnants which are taken up mainly in the liver via recognition of their ApoE portion. The synthesis, maturation, use and recycling of VLDL particles follows the very same pathway, but starts in the liver and is using the ApoB-100 protein as its structure forming unit. Again, ApoE mediates the eventual uptake and recycling of the VLDL-remnants, the so-called IDL particles. (see also http://en.wikipedia.org/wiki/Lipoprotein)

ApoE shares structural homology to the apolipoproteins A and C in that they all comprise amphipathic tandem repeats of 11 amino acids. Crystallographic data confirm the existence of extended amphipathic helical structures for ApoA-I and and ApoE fragment and also reveal a mixed charge organization on the polar face of these helices. These data are publicly available from the RCSB Protein Data Bank (http://www.rcsb.orp/pdb/home/home.do) and entry 1AV1.pdb gives the protein structure of ApoA-I. The amino acids 129 to 166 of 1Ipe.pdb represent the LDL-receptor binding fragment of ApoE. In contrast to their overall similarity, the three apolipoproteins display specific deviations when their amino acid composition is analyzed. In ApoE, arginine is the prevailing cationic amino acid in the tandem repeats. In contrast, ApoA has equal amounts of lysine and arginine while ApoC has an excess of lysine residues.

**Table 1: Analysis of the amino acid composition in tandem repeats of related apolipoproteins. Sequence data were obtained from Swiss-Prot (http://www.expasy.ch/sprot/sprot-top.html).**

| Sequence | ApoAI | ApoE | ApoC-II |
|---|---|---|---|
| SwissProt Entry | P02647 | P02649 | P02655 |
| Endpoints | 68 - 267 | 80-255 | 23-101 |
| lenght | 199 | 175 | 78 |
| IP | 5,55 | 9,16 | 4,66 |
| # of lysine | 18 | 8 | 6 |
| # of arginine | 14 | 25 | 1 |
| # of histidine | 5 | 1 | 0 |
| # of aspartic acid | 10 | 8 | 4 |
| # of glutamic acid | 28 | 22 | 7 |
| | ApoAI | ApoE | ApoC-II |
| Lysine (%) | 9% | 5% | 8% |
| Arginine (%) | 7% | 14% | 1% |
| Histidine (%) | 3% | 1% | 0% |
| Aspartic acid (%) | 5% | 5% | 5% |
| Glutamic acid (%) | 14% | 13% | 9% |

In summary, the polar surface of natural lipoproteins is covered with apolipoproteins out of which ApoE is a common binding motif for the cellular uptake of these particles. The water-exposed portions of ApoE represent a mosaic of anionic and cationic charges, wherein the anionic charges are created from the free carboxyl termini of aspartic and glutamic acid residues. The cationic charges comprise a mixture of amino and guanido groups with a very few imidazols being present.

In order to emulate the recognition pattern of the ApoE binding cassette on the surface of liposomes, different alternatives can be followed. It is well possible to synthesize ApoE peptide fragment and graft such peptides on the surface of liposomes. This has been demonstrated by Mims et al., J Biol. Chem. 269, 20539 (1994); Rensen et al., Mol Pharmacol. 52, 445 (1997); Rensen et al., J. Lipid Res. 38, 1070 (1997); Sauer et al., Biochemistry 44, 2021 (2005) or Versluis et al., J Pharmacol. Exp. Ther 289, 1 (1999). However, the high cost associated with peptide synthesis and derivatization call for alternative approaches.

A direct presentation of the required charged moieties using mixtures of different charged lipids, potentially further comprising neutral lipids would yield a much simpler structure and eliminate the needs for costly peptide production and derivatization. A considerable challenge of such approach is the planar diffusion of the charged groups within the lipid bilayer; it was therefore unclear whether the affinity of such a less organized assembly would effectively compete with the affinities provided by the authentic lipoproteins.

### Lipid Chemistry

Anionic lipids comprising carboxyl moieties in their polar head groups are well know to the skilled artisan. Instant examples can be selected from the structures (1) - (4) below, 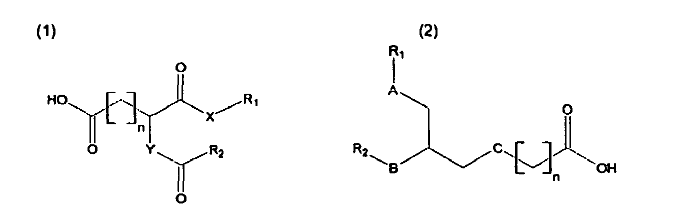 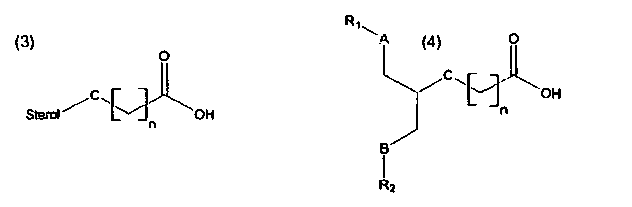 wherein n is any number between 0 and 29, X and Y are independently from each other -O- or -NH- and R1 and R2 are independently from each other an alkyl, alkenyl or alkinyl moieties having between 8 and 24 carbon atoms and 0, 1 or 2 unsaturated bonds, A, B or C are independently from each other -O-, -NH-, -C(O)-O-, -O-C(O)-, -C(O)-NH-, -NH-C(O)-, -O-C(O)-NH- or -NH-C(O)-O- and "sterol" can be a cholesterol attached via its C3 atom.

The list below provides further specific examples of lipids carrying a carboxyl group.
- CHEMS: Cholesterolhemisuccinate
- Chol-COOH or Chol-C1: Cholesteryl-3-carboxylic acid
- Chol-C2: Cholesterolhemioxalate
- Chol-C3: Cholesterolhemimalonate
- Chol-C3N: N-(Cholesteryl-oxycarbonyl)glycine
- Chol-C5: Cholesterolhemiglutarate
- Chol-C6: Cholesterolhemiadipate
- Chol-C7: Cholesterolhemipimelate
- Chol-C8: Cholesterolhemisuberate
- Chol-C12: Cholesterolhemidodecane dicarboxylic acid
- Chol-C13N: 12-Cholesteryloxycarbonylaminododecanoic acid

Cholesterolhemidicarboxylic acids and Cholesteryloxycarbonylaminocarboxylic acids of following general formula: 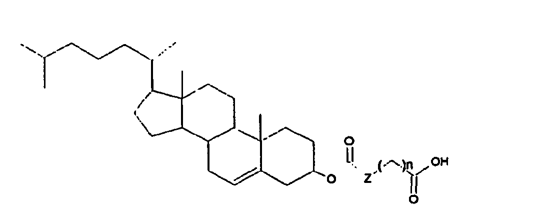 wherein Z is C or -NH- and n is any number between 0 and 29.
- DGS or DG-Succ: Diacylglycerolhemisuccinate (unspecified membrane anchor)
- DOGS or DOG-Succ: Dioleoylglycerolhemisuccinate
- DMGS or DMG-Succ: Dimyristoylglycerolhemisuccinate
- DPGS or DPG-Succ: Dipalmitoylglycerolhemisuccinate
- DSGS or DSG-Succ: Distearoylglycerolhemisuccinate
- POGS or POG-Succ: 1-Palmitoyl-2-oleoylglycerol-hemisuccinate
- DOGM: Dioleoylglycerolhemimalonate
- DOGG: Dioleoylglycerolhemiglutarate
- DOGA: Dioleoylglycerolhemiadipate
- DMGM: Dimyristoylglycerolhemimalonate
- DMGG: Dimyristoylglycerolhemiglutarate
- DMGA: Dimyristoylglycerolhemiadipate
- DOAS: 4-{(2,3-Dioleoyl-propyl)amino}-4-oxobutanoic acid
- DOAM: 3-{(2,3-Dioleoyl-propyl)amino}-3-oxopropanoic acid
- DOAG: 5-{(2,3-Dioleoyl-propyl)amino}-5-oxopentanoic acid
- DOAA: 6-{(2,3-Dioleoyl-propyl)amino}-6-oxohexanoic acid
- DMAS: 4-{(2,3-Dimyristoyl-propyl)amino}-4-oxobutanoic acid
- DMAM: 3-{(2,3-Dimyristoyl-propyl)amino}-3-oxopropanoic acid
- DMAG: 5-{(2,3-Dimyristoyl-propyl)amino}-5-oxopentanoic acid
- DMAA: 6-{(2,3-Dimyristoyl-propyl)amino}-6-oxohexanoic acid
- DOP: 2,3-Dioleoyl-propanoic acid
- DOB: 3,4-Dioleoyl-butanoic acid
- DOS: 5,6-Dioleoyl-hexanoic acid
- DOM: 4,5-Dioleoyl-pentanoic acid
- DOG: 6,7-Dioleoyl-heptanoic acid
- DOA: 7,8-Dioleoyl-octanoic acid
- DMP: 2,3-Dimyristoyl-propanoic acid
- DMB: 3,4-Dimyristoyl-butanoic acid
- DMS: 5,6-Dimyristoyl-hexanoic acid
- DMM: 4,5-Dimyristoyl-pentanoic acid
- DMG: 6,7-Dimyristoyl-heptanoic acid
- DMA: 7,8-Dimyristoyl-octanoic acid
- DOG-GluA: Dioleoylglycerol-glucoronic acid (1- or 4-linked)
- DMG-GluA: Dimyristoylglycerol-glucoronic acid (1- or 4-linked)
- DO-cHA: Dioleoylglycerolhemicyclohexane-1,4-dicarboxylic acid
- DM-cHA: Dimyristoylglycerolhemicyclohexane-1,4-dicarboxylic acid
- PS: Phosphatidylserine (unspecified membrane anchor)
- DOPS: Dioleoylphosphatidylserine
- DPPS: Dipalmitoylphosphatidylserine
- MA: Myristic Acid
- PA: Palmitic Acid
- OA: Oleic Acid
- LA: Linoleic Acid
- SA: Stearic Acid
- NA: Nervonic Acid
- BA: Behenic Acid

Any dialkyl derivatives of the anionic lipids comprising diacyl groups listed above are also within the scope of the present invention.

Preferred anionic lipids having a carboxyl group can be selected from the group of Chol-C1 to Chol-C16 including all its homologues, in particular CHEMS. Also preferred are the anionic lipids DMGS, DPGS, DSGS, DOGS, POGS.

Cationic lipids for the use with this invention are also known to the skilled artisan and comprise, amongst others, the structures (5) - (8), 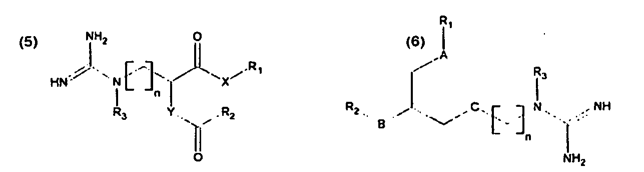 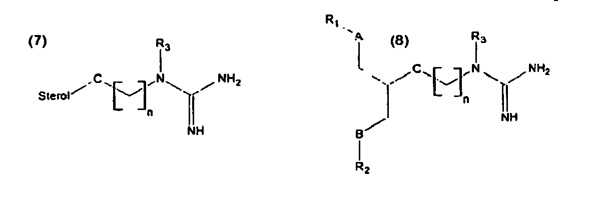 wherein n is any number between 0 and 29, X and Y are independently from each other -O- or -NH- and R1, and R2 are independently from each other an alkyl, alkenyl or alkinyl moieties having between 8 and 24 carbon atoms and 0, 1 or 2 unsaturated bonds, R3 is H or an alkyl, alkenyl, alkinyl, cycloalkyl or aryl moiety comprising between 0 and 8 carbon atoms and 0, 1, 2 or 3 unsaturated bonds A, B or C are independently from each other -O-, -NH-, -C(O)-O-, -O-C(O)-, -C(O)-NH-, - NH-C(O)-, -O-C(O)-NH- or -NH-C(O)-O- and "sterol" can be a cholesterol attached via its C3 atom.

Some of these lipids have been presented earlier in the literature, for example in WO91/16024, WO97/43363, WO98/05678, WO01/55098, WO28137758, in EP 0685234 or in US 5965434.

It is apparent from the analysis above that in addition to the guanido groups, the cationic head groups on the liposome surface may comprise other charged moieties.

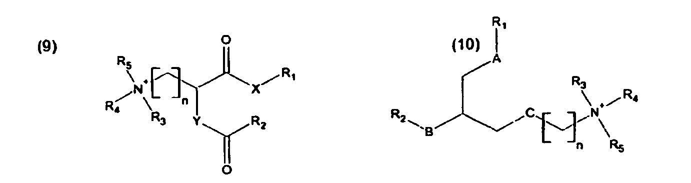

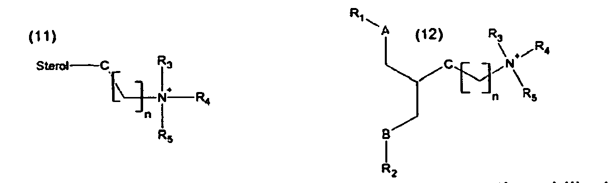

Amongst said other cationic lipids known to the skilled artisan, such lipids may carry primary, secondary, tertiary or quarternized amino groups as in the structures (9)-(12) below:
wherein n is any number between 0 and 29, X and Y are independently from each other -O- or -NH- and R1, and R2 are independently from each other an alkyl, alkenyl or alkinyl moieties having between 8 and 24 carbon atoms and 0, 1 or 2 unsaturated bonds, R3, R4 and R5 are independently from each other H or an alkyl, alkenyl, alkinyl, cycloalkyl or aryl moiety comprising between 0 and 8 carbon atoms and 0, 1, 2 or 3 unsaturated bonds and R5 may be absent, A, B or C are independently from each other -O-, -NH-, -C(O)-O-, -O-C(O)-, -C(O)-NH-, -NH-C(O)-, -O-C(O)-NH- or -NH-C(O)-O- and "sterol" can be a cholesterol attached via its C3 atom.

### Lipid Assemblies

The components mentioned above can be assembled in various structures known to the skilled artisan. This can be liposomes comprising one or a number of individual bilayers, other supramolecular lipid assemblies, vesicles having a sizeable interior volume that provides an aqueous phase. It also can be emulsion droplets or structures in the form of lipoplex assemblies, the latter in many embodiments comprising electrostatic complexes between the lipids and nucleic acids.

It is also known in the art that neutral lipids may be part of these various lipid assemblies. Neutral lipids such as cholesterol or zwitterionic lipids such as phosphatidylethanolamine, phosphatidylcholine, sphingomyelin and the like are often used to modulate the properties of these assemblies. WO08/043575 gives specific guidance for the types and use of neutral lipids and their impact on the fusogenicity and stability of liposomes.

In a specific embodiment of this invention, the cationic guanido lipid PONA (palmitoyloleoyl-nor-arginine, structure 13) was combined with various lipid anions such as CHEMS or DMGS. The ratios of the cationic and anionic lipids were varied in a systematic way and the resulting binary mixtures optionally were further supplied with 20 mol% cholesterol. The individual lipid mixtures were transformed into liposomes and used for the encapsulation of an active and control siRNA. When tested on HeLa cells, efficient and specific inhibition of the cell viability was observed for most of the tested formulations, as demonstrated in example 5. The activity was not or only marginally affected by the presence of human or mouse serum.

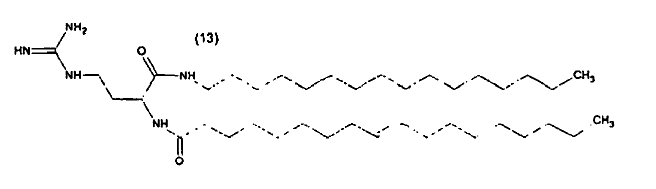

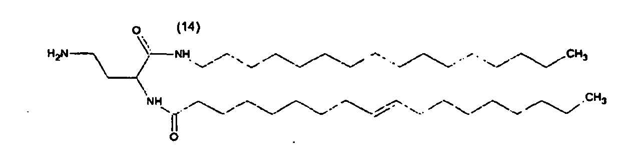

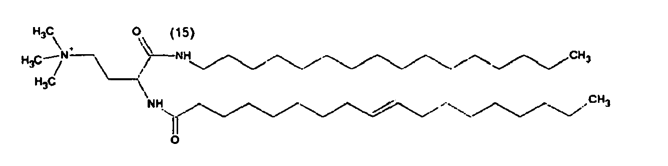

In example 6, the anionic lipid CHEMS was combined with derivatives of PONA, wherein the guanido moiety was substituted by an amino group (PONamine) or an quarternized ammonium group (PONammonium) as shown in the structures (14) and (15). Again, the ratios between the anionic and cationic lipid components were systematically varied and 20% cholesterol was present in all lipid mixtures. The material was formulated into liposomes and used for the encapsulation of active and control siRNA. When tested on HeLa cells, efficient and specific inhibition of the cell viability was observed for all formulations comprising a molar excess of the cationic lipids. For mixtures comprising higher molar amounts of the anionic lipid CHEMS, the best activity was observed in combinations with PONA, while PONamine: CHEMS combinations were only effective in some cases. The PONammonium:CHEMS combinations were not effective when an excess of the anionic lipid was used.

Moreover, out of the mixtures comprising an excess of the anionic lipid CHEMS, the transfection activity of the PONA:CHEMS combinations was only marginally affected by the presence of human or mouse serum, while the activity of PONamine:CHEMS combinations was completed suppressed in the presence of mouse serum. The PONammonium formulations remained inactive in the presence of sera.

The combined data support a preferred uptake of lipid combinations comprising guanido lipids in combination with carboxyl lipids. This may relate to the mechanistic considerations made above. The constant and high activity of the formulations having an excess of the cationic lipid component may be due to electrostatic interaction between these particles and the cell surface, which however is unspecific. In line with this view is the fact that the activity of the cationic formulations did neither depend on the nature of the anionic or cationic lipid.

To identify further preferred embodiments of this invention, the guanido lipid PONA was combined with CHEMS, DMGS or DOGS. Again, a systematic variation of the ratios of both the anionic and cationic lipid compound in the respective binary mixtures was done and the formulations were further supplied with 0, 20 or 40 mol% of cholesterol. When tested as above, the great majority of the formulations were active in inhibiting the cell proliferation of HeLa cells with an IC50 being lower than 6nM (see example 7). A comparison between the concentrations needed for the efficacy of the active and inactive siRNA, however, revealed substantial differences between the formulations. A measure for such comparison is the ratio between the IC50 values for both siRNA's, here expressed as SCR/PLK ratio. Only selected formulations reach values significantly higher than 5. Even more preferred formulations have SCR/PLK>=10. Strinkingly, all these preferred formulations can be characterized by their ratio between the anionic and cationic lipid component, which here is lower than 1.

This invention identifies specific lipid head group chemistry as critical for the uptake into certain cells in the presence of otherwise inhibitory sera. With preference, combinations of anionic lipids comprising carboxyl groups and cationic lipids comprising guanido moieties result in the desired properties.

Amongst liposomes with such characteristics, those having a molar excess of the anionic over the cationic lipid have particular high safety features in that the viability of the tested cells was not affected by liposomes formed from these materials, but sequestering an unrelated siRNA.

### Cargoes for the liposomes of this invention

As presented above the liposomes or lipid assemblies of this invention encapsulate at least one active agent, this can be extended to numerous other embodiments. Said active agent may comprise a drug. In some embodiments said active agent may comprises a nucleic acid.

Without being limited to such use, the liposomes or lipid assemblies described in the present invention are well suited for use as carriers for nucleic acid-based drugs such for example as oligonucleotides, polynucleotides and DNA plasmids. These drugs are classified into nucleic acids that encode one or more specific sequences for proteins, polypeptides or RNAs and into oligonucleotides that can specifically regulate protein expression levels or affect the protein structure through inter alia interference with splicing and artificial truncation.

In some embodiments of the present invention, therefore, the nucleic acid-based therapeutic may comprise a nucleic acid that is capable of being transcribed in a vertebrate cell into one or more RNAs, which RNAs may be mRNAs, shRNAs, miRNAs or ribozymes, wherein such mRNAs code for one or more proteins or polypeptides. Such nucleic acid therapeutics may be circular DNA plasmids, linear DNA constructs, like MIDGE vectors (Minimalistic Immunogenically Defined Gene Expression) as disclosed in WO 98/21322 or DE 19753182, or mRNAs ready for translation (e.g., EP 1392341).

In other embodiments of the invention, oligonucleotides may be used that can target existing intracellular nucleic acids or proteins. Said nucleic acids may code for a specific gene, such that said oligonucleotide is adapted to attenuate or modulate transcription, modify the processing of the transcript or otherwise interfere with the expression of the protein. The term "target nucleic acid" encompasses DNA encoding a specific gene, as well as all RNAs derived from such DNA, being premRNA or mRNA. A specific hybridisation between the target nucleic acid and one or more oligonucleotides directed against such sequences may result in an inhibition or modulation of protein expression. To achieve such specific targeting, the oligonucleotide should suitably comprise a continuous stretch of nucleotides that is substantially complementary to the sequence of the target nucleic acid.

Oligonucleotides fulfilling the abovementioned criteria may be built with a number of different chemistries and topologies. The oligonucleotides may comprise naturally occurring or modified nucleosides comprising but not limited to DNA, RNA, locked nucleic acids (LNA's), unlocked nucleic acids (UNA's), 2'O-methyl RNA (2'Ome), 2' O-methoxyethyl RNA (2'MOE) in their phosphate or phosphothioate forms or Morpholinos or peptide nucleic acids (PNA's). Oligonucleotides may be single stranded or double stranded.

Oligonucleotides are polyanionic structures having 8-60 charges. In most cases these structures are polymers comprising nucleotides. The present invention is not limited to a particular mechanism of action of the oligonucleotides and an understanding of the mechanism is not necessary to practice the present invention. The mechanisms of action of oligonucleotides may vary and might comprise inter alia effects on splicing, transcription, nuclear-cytoplasmic transport and translation.

In a preferred embodiment of the invention single stranded oligonucleotides may be used, including, but not limited to DNA-based oligonucleotides, locked nucleic acids, 2'-modified oligonucleotides and others, commonly known as antisense oligonucleotides. Backbone or base or sugar modifications may include, but are not limited to, Phosphothioate DNA (PTO), 2'O-methyl RNA (2'Ome), 2'Fluoro RNA (2'F), 2' O- methoxyethyl-RNA (2'MOE), peptide nucleic acids (PNA), N3'-P5' phosphoamidates (NP), 2'fluoroarabino nucleic acids (FANA), locked nucleic acids (LNA), unlocked nucleic acids (UNA), Morpholine phosphoamidate (Morpholino), Cyclohexene nucleic acid (CeNA), tricyclo-DNA (tcDNA) and others. Moreover, mixed chemistries are known in the art, being constructed from more than a single nucleotide species as copolymers, block-copolymers or gapmers or in other arrangements.

In addition to the aforementioned oligonucleotides, protein expression can also be inhibited using double stranded RNA molecules containing the complementary sequence motifs. Such RNA molecules are known as siRNA molecules in the art (e.g., WO 99/32619 or WO 02/055693). Other siRNAs comprise single stranded siRNAs or double stranded siRNAs having one non-continuous strand. Again, various chemistries were adapted to this class of oligonucleotides. Also, DNA / RNA hybrid systems are known in the art. Other varieties of siRNA's comprise three-stranded constructs wherein two smaller strand hydridize to one common longer strand, the so-called meroduplex or sisiRNA's having nicks or gaps in their architecture.

In another embodiment of the present invention, decoy oligonucleotides can be used. These double stranded DNA molecules and chemical modifications thereof do not target nucleic acids but transcription factors. This means that decoy oligonucleotides bind sequence-specific DNA-binding proteins and interfere with the transcription (e.g., Cho-Chung, et al. in Curr. Opin. Mol. Ther., 1999).

In a further embodiment of the invention oligonucleotides that may influence transcription by hybridizing under physiological conditions to the promoter region of a gene may be used. Again various chemistries may adapt to this class of oligonucleotides.

In a still further alternative of the invention, DNAzymes may be used. DNAzymes are single-stranded oligonucleotides and chemical modifications therof with enzymatic activity. Typical DNAzymes, known as the "10-23" model, are capable of cleaving single-stranded RNA at specific sites under physiological conditions. The 10-23 model of DNAzymes has a catalytic domain of 15 highly conserved deoxyribonucleotides, flanked by 2 substrate-recognition domains complementary to a target sequence on the RNA. Cleavage of the target mRNAs may result in their destruction and the DNAzymes recycle and cleave multiple substrates.

In yet another embodiment of the invention ribozymes can be used. Ribozymes are single-stranded oligoribonucleotides and chemical modifications thereof with enzymatic activity. They can be operationally divided into two components, a conserved stem-loop structure forming the catalytic core and flanking sequences which are reverse complementary to sequences surrounding the target site in a given RNA transcript. Flanking sequences may confer specificity and may generally constitute 14-16 nt in total, extending on both sides of the target site selected.

In a still further embodiment of the invention aptamers may be used to target proteins. Aptamers are macromolecules composed of nucleic acids, such as RNA or DNA, and chemical modifications thereof that bind tightly to a specific molecular target and are typically 15-60 nt long. The chain of nucleotides may form intramolecular interactions that fold the molecule into a complex three-dimensional shape. The shape of the aptamer allows it to bind tightly against the surface of its target molecule including but not limited to acidic proteins, basic proteins, membrane proteins, transcription factors and enzymes. Binding of aptamer molecules may influence the function of a target molecule.

All of the above-mentioned oligonucleotides may vary in length between as little as 5 or 10, preferably 15 and even more preferably 18, and 50, preferably 30 and more preferably 25, nucleotides per strand. More specifically, the oligonucleotides may be antisense oligonucleotides of 8 to 50 nucleotides length that catalyze RNAseH mediated degradation of their target sequence or block translation or re-direct splicing or act as antogomirs; they may be siRNAs of 15 to 30 basepairs length; they may further represent decoy oligonucleotides of 15 to 30 basepairs length; can be complementary oligonucleotides influencing the transcription of genomic DNA of 15 to 30 nucleotides length; they might further represent DNAzymes of 25 to 50 nucleotides length or ribozymes of 25 to 50 nucleotides length or aptamers of 15 to 60 nucleotides length. Such subclasses of oligonucleotides are often functionally defined and can be identical or different or share some, but not all features of their chemical nature or architecture without substantially affecting the teachings of this invention. The fit between the oligonucleotide and the target sequence is preferably perfect with each base of the oligonucleotide forming a base pair with its complementary base on the target nucleic acid over a continuous stretch of the abovementioned number of oligonucleotides. The pair of sequences may contain one or more mismatches within the said continuous stretch of base pairs, although this is less preferred. In general the type and chemical composition of such nucleic acids is of little impact for the performance of the inventive liposomes as vehicles be it in vivo or in vitro and the skilled artisan may find other types of oligonucleotides or nucleic acids suitable for combination with the inventive amphoteric liposomes.

In certain aspects and as demonstrated within this invention, the liposomes according to the present invention are useful to transfect cells *in vitro, in vivo* or *ex vivo.*

### Examples

The teachings of this invention may be better understood with the consideration of the following examples. However, these examples should by no means limit the teachings of this invention.

### Example 1 - Liposome production, characterization and encapsulation of siRNA

Liposomes were prepared using methods as disclosed in WO07/107304. More specifically, lipids were dissolved in isopropanol and liposomes were produced by adding siRNA solution in NaAc 20mM, Sucrose 300 mM, pH 4.0 (pH adjusted with HAc) to the alcoholic lipid mix, resulting in a final alcohol concentration of 30%. The formed liposomal suspensions were shifted to pH 7.5 with twice the volume of Na₂HPO₄ 136mM, NaCl 100mM (pH 9), resulting in a final lipid concentration of 3 mM and a final isopropanol concentration of 10%.

Liposomes were characterized with respect to their particle size using dynamic light scattering (MALVERN 3000HSA).

Active siRNA: 21 mer blunt ended targeting mouse and human PLK-1 mRNA as in Haupenthal et al., Int J Cancer (2007), 121:206-210.

Control siRNA (SCR): 21 mer from the same source.

### Example 2 - General Cell Culture and Proliferation Assay

HeLa cells were obtained from DSMZ (German Collection of Micro Organism and Cell Cultures) and maintained in DMEM (Gibco-Invitrogen) and supplemented with 10% FCS. The cells were plated at a density of 2.5*10⁴ cells/ml and cultivated in 100 µl medium at 37 °C under 5% CO₂. After 16 h the liposomes containing siRNA were diluted and 10 µl were added to the cells to yield final concentrations between 0,4 to 100 nM Plk1 or scrambled siRNA; 10µl dilution buffer were also added to untreated cells and into wells without cells. Cell culture dishes were incubated for 72 h hours at 37 °C under 5% CO₂.

Cell proliferation/viability was determined by using the CellTiter-Blue Cell Viability assay (Promega, US) according to the instructions of the supplier.

### Example 3 - Inhibition of transfection by sera

Liposomes from DODAP:DMGS:Cholesterol (24:36:40 mol%) were loaded with active and control siRNA as above and 25µl of the liposomes were incubated with 75µl sera from different species (SIGMA-Aldrich) for 30min. Following that, liposomes were added to the cells, incubation was continued for 72h and cell viability was determined as above.

When incubated without serum, administration of the active siRNA results in a strong inhibition of cell proliferation. As demonstrated in the table 2 below, this process is inhibited by the addition of sera.

**Table 2: Inhibition of cellular transfection by sera of different origin.**

| siRNA type | siRNA concentration | Serum | Cell viability (%) |
|---|---|---|---|
| PLK1 | 50nM | no | 7 |
| PLK1 | 50nM | Human | 98 |
| PLK1 | 50nM | Hamster | 80 |
| PLK1 | 50nM | Rat | 108 |
| PLK1 | 50nM | Mouse | 102 |
| No | No | No | 100 |

### Example 4 - Inhibition is lipoprotein dependent

Liposomes as in example 3 were incubated with human serum devoid of certain complement factors or lipoproteins (SIGMA-Aldrich) as above and analyzed for their ability to mediated the RNAi effect on HeLa cells.

As demonstrated in table 3, the efficacy of transfection can be restored by a depletion of lipoproteins. Removal of complement factors was ineffective.

**Table 3: Restoration of cellular transfection in sera being deficient of various factors.**

| siRNA type | siRNA concentration | Serum | Cell viability (%) |
|---|---|---|---|
| PLK1 | 50nM | no | 7 |
| PLK1 | 50nM | Human, complete | 98 |
| PLK1 | 50nM | Human, no C3 complement factor | 91 |
| PLK1 | 50nM | Human, no C9 complement factor | 98 |
| PLK1 | 50nM | Human, lipoprotein deficient | 18 |
| No | No | No | 100 |

### Example 5 - Serum resistant transfection

A series of liposomes was constructed from PONA:Anionic Lipid:Cholesterol (x:y:20 mol%) and loaded with active and control siRNA as in example 1. Within that series, the ratio between the cationic component PONA and the anionic lipids CHEMS or DMGS was systematically varied between 0.33 and 2 as indicated in the table. Liposomes having a ratio of the cationic : anionic lipid of 1 or greater were further supplied with 2mol% DMPE-PEG2000 (Nippon Oils and Fats) to avoid aggregation of the particles. This modification is indicated by a "+" in the table.

HeLa cells were grown and maintained as in example 2 and sera of human or mice origin (SIGMA-Aldrich) was added directly to the cells for 120min. Following that, the liposomes were added to the cells in concentrations between 50pM and 50nM, incubation was continued for 72h and cell viability was determined as above. The efficacy of transfection is expressed here as IC50, the concentration needed to inhibited cell proliferation by 50%. Low IC50 values therefore represent highly effective transfection.

It becomes apparent from the results in the table 4, that the addition of sera only marginally affects the transfection of siRNA mediated by the liposomes of the example. Some inhibition is still observed for liposomes from PONA:CHEMS comprising low amounts of the anionic lipid (ratios 0.33 and 0.5, particular strong inhibition with mouse serum).

**Table 4: Efficacy of transfection of liposomes comprising guanido moieties in the presence of sera.**

| | CHEMS | | | | | | | |
|---|---|---|---|---|---|---|---|---|
| Ratio cationic/anionic lipid | 0,33 | 0,50 | 0,67 | 0,82 | 1 + | 1,22+ | 1,5+ | 2+ |
| No Serum | 38,54 | 1,21 | 0,40 | 0,56 | 1,83 | 1,61 | 0,70 | 1,42 |
| Human Serum | 199,00 | 2,10 | 0,62 | 1,13 | 2,16 | 1,92 | 1,70 | 1,83 |
| Mouse Serum | 199,00 | 50,00 | 1,56 | 1,94 | 2,47 | 1,90 | 0,76 | 1,44 |
| | | | | | | | | |

| | DMGS | | | | | | | |
|---|---|---|---|---|---|---|---|---|
| No Serum | | | 0,23 | 0,54 | 0,01 | 0,01 | | |
| Human Serum | | | 1,50 | 2,39 | 2,88 | 2,21 | | |
| Mouse Serum | | | 0,67 | 0,69 | 1,41 | 1,81 | | |

### Example 6 - Criticality of the guanido head group

Series of liposomes having systematically varied ratios between the cationic and anionic lipid components were produced and loaded with siRNA as in example 5. The cationic lipid components were PONA, PONamine and PONammonium, the anionic lipid was CHEMS and the cholesterol content was fixed to 20 mol%. Liposomes having a ratio of the cationic : anionic lipid of 1 or greater were further supplied with 2mol% DMPE-PEG2000 (Nippon Oils and Fats) to avoid aggregation of the particles. This modification is indicated by a "+" in the table.

HeLa cells were grown and maintained as in example 2 and sera of human or mice origin (SIGMA-Aldrich) was added directly to the cells for 120min. Following that, the liposomes were added to the cells in concentrations between 50pM and 50nM, incubation was continued for 72h and cell viability was determined as above. The efficacy of transfection is expressed here as IC50 as in example 5.

It becomes apparent from the data in table 5, that only PONA, but neither PONamine and even less so PONammonium mediates the transfection of HeLa cells in the presence of serum. This is most striking in the case of mouse serum, which inhibits the transfection more aggressively. An excess of the cationic lipid components to some extent compensate the serum mediated loss of activity, but may be due to unspecific electrostatic adsorption of these liposomes to the cells.

**Table 5: Criticality of the guanido head group for the serum resistant transfection of cells.**

| | PONA | | | | | | | |
|---|---|---|---|---|---|---|---|---|
| C/A ratio | 0,33 | 0,5 | 0,67 | 0,82 | 1+ | 1,22+ | 1,5+ | 2+ |
| no serum | 42,9 | 1,8 | 0,6 | 1,0 | 4,1 | 5,4 | 2,4 | 6,8 |
| human serum | 80,0 | 2,5 | 2,2 | 2,0 | 1,8 | 2,8 | 6,2 | 5,2 |
| mouse serum | 80,0 | 31,1 | 55,0 | 5,7 | 2,1 | 5,3 | 8,1 | 7,5 |
| | | | | | | | | |

| | PONamine | | | | | | | |
|---|---|---|---|---|---|---|---|---|
| C/A ratio | 0,33 | 0,5 | 0,67 | 0,82 | 1 + | 1,22+ | 1,5+ | 2+ |
| no serum | 3,1 | 65,0 | 7,5 | 100,0 | 3,0 | 5,2 | 3,0 | 2,5 |
| human serum | 100,0 | 55,0 | 11,9 | 100,0 | 2,2 | 2,8 | 6,1 | 5,1 |
| mouse serum | 70,0 | 100,0 | 100,0 | 100,0 | 75,0 | 70,0 | 39,3 | 8,7 |
| | | | | | | | | |

| | PONammonium | | | | | | | |
|---|---|---|---|---|---|---|---|---|
| C/A ratio | 0,33 | 0,5 | 0,67 | 0,82 | 1 + | 1,22+ | 1,5+ | 2+ |
| no serum | 80,0 | 100,0 | 90,0 | 90,0 | 65,0 | 9,5 | 9,5 | 5,2 |
| human serum | 95,0 | 90,0 | 90,0 | 80,0 | 90,0 | 11,8 | 12,4 | 15,7 |
| mouse serum | 85,0 | 100,0 | 100,0 | 100,0 | 100,0 | 90,0 | 75,0 | 55,0 |

### Example 7 - Optimization of the liposome composition

Series of liposomes having systematically varied ratios between the cationic and anionic lipid components were produced and loaded with siRNA as in example 5. The cationic lipid component was PONA, the anionic lipids were CHEMS, DMGS or DOGS and the cholesterol content was varied between 0 and 40mol%. Liposomes having a ratio of the cationic : anionic lipid of 1 or greater but also some of the other liposomes were further supplied with 2mol% DMPE-PEG2000 (Nippon Oils and Fats) to avoid aggregation of the particles. This modification is indicated by a "+" in the table.

HeLa cells were grown and maintained as in example 2 and liposomes were added to the cells in concentrations between 6nM and 200nM, incubation was continued for 72h and cell viability was determined as above. The efficacy of transfection is expressed here as IC50 as in the examples above. In addition, the IC50 was determined for the liposomes carrying the inactive siRNA (SCR) and the ratio between IC50(SCR) and IC50(PLK1) was determined. A high value for this parameter indicates a very specific inhibition of the cellular viability by the PLK1 siRNA, low unspecific effects contributed by the carrier and low levels of cytotoxicity in general.

**Table 6: Optimization results for CHEMS. Lowest and highes detectable IC50 values are 6 and 200nM, respectively.**

| PLK | 0,33 | 0,33+ | 0,5 | 0,5+ | 0,67 | 0,67+ | 0,82 | 0,82+ | 1 + | 1,22+ | 1,5+ | 2+ |
|---|---|---|---|---|---|---|---|---|---|---|---|---|
| 0% Chol | 44 | 77 | 6 | 6 | 6 | 6 | 6 | 6 | 6 | 6 | 6 | 6 |
| 20% Chol | 54 | 79 | 6 | 6 | 6 | 6 | 6 | 6 | 6 | 6 | 6 | 6 |
| 40% Chol | 67 | 94 | 6 | 6 | 6 | 6 | 6 | 6 | 6 | 6 | 6 | 6 |
| | | | | | | | | | | | | |

| SCR | 0,33 | 0,33+ | 0,5 | 0,5+ | 0,67 | 0,67+ | 0,82 | 0,82+ | 1+ | 1,22+ | 1,5+ | 2+ |
|---|---|---|---|---|---|---|---|---|---|---|---|---|
| 0% Chol | 90 | 86 | 113 | 152 | 23 | 200 | 16 | 21 | 15 | 16 | 14 | 11 |
| 20% Chol | 73 | 90 | 109 | 128 | 200 | 200 | 26 | 23 | 21 | 11 | 16 | 10 |
| 40% Chol | 94 | 117 | 198 | 200 | 200 | 200 | 6 | 6 | 30 | 14 | 27 | 12 |
| | | | | | | | | | | | | |

| SCR/PLK | 0,33 | 0,33+ | 0,5 | 0,5+ | 0,67 | 0,67+ | 0,82 | 0,82+ | 1+ | 1,22+ | 1,5+ | 2+ |
|---|---|---|---|---|---|---|---|---|---|---|---|---|
| 0% Chol | 2,05 | 1,12 | 18,86 | 25,33 | 3,81 | 83,33 | 2,60 | 3,52 | 2,50 | 2,68 | 2,30 | 1,84 |
| 20% Chol | 1,37 | 1,14 | 18,10 | 21,39 | 83,33 | 83,33 | 4,26 | 3,77 | 3,45 | 1,84 | 2,65 | 1,69 |
| 40% Chol | 1,40 | 1,24 | 32,96 | 83,33 | 83,33 | 83,33 | 1,00 | 1,00 | 5,00 | 2,39 | 4,48 | 1,97 |

**Table 7: Optimization results for CHEMS. Lowest and highes detectable IC50 values are 6 and 200nM, respectively.**

| PLK | 0,33 | 0,5 | 0,67 | 0,82 | 1+ | 1,22+ | 1,5+ | 2+ |
|---|---|---|---|---|---|---|---|---|
| 0% Chol | 98 | 200 | 200 | 188 | 6 | 6 | 6 | 6 |
| 20% Chol | 6 | 6 | 6 | 6 | 6 | 6 | 6 | 6 |
| 40% Chol | 6 | 6 | 6 | 6 | 6 | 6 | 6 | 6 |
| | | | | | | | | |

| SCR | 0,33 | 0,5 | 0,67 | 0,82 | 1+ | 1,22+ | 1,5+ | 2+ |
|---|---|---|---|---|---|---|---|---|
| 0% Chol | 200 | 200 | 200 | 158 | 14 | 6 | 10 | 14 |
| 20% Chol | 200 | 54 | 8 | 8 | 13 | 9 | 9 | 10 |
| 40% Chol | 155 | 23 | 11 | 6 | 6 | 14 | 9 | 12 |
| | | | | | | | | |

| SCR/PLK | 0,33 | 0,5 | 0,67 | 0,82 | 1+ | 1,22+ | 1,5+ | 2+ |
|---|---|---|---|---|---|---|---|---|
| 0% Chol | 5,11 | no effect | no effect | 0,84 | 2,26 | 1,00 | 1,66 | 2,36 |
| 20% Chol | 83,33 | 9,01 | 1,27 | 1,26 | 2,20 | 1,55 | 1,45 | 1,69 |
| 40% Chol | 25,85 | 3,90 | 1,83 | 1,00 | 1,00 | 2,27 | 1,54 | 1,97 |

**Table 8: Optimization results for CHEMS. Lowest and highes detectable IC50 values are 6 and 200nM, respectively.**

| PLK | 0,33 | 0,5 | 0,67 | 0,82 | 1+ | 1,22+ | 1,5+ | 2+ |
|---|---|---|---|---|---|---|---|---|
| 0% Chol | 200 | 200 | 200 | 200 | 6 | 6 | 6 | 6 |
| 20% Chol | 22 | 200 | 200 | 200 | 6 | 6 | 6 | 6 |
| 40% Chol | 6 | 170 | 200 | 200 | 6 | 6 | 6 | 6 |
| | | | | | | | | |

| SCR | 0,33 | 0,5 | 0,67 | 0,82 | 1+ | 1,22+ | 1,5+ | 2+ |
|---|---|---|---|---|---|---|---|---|
| 0% Chol | 200 | 200 | 200 | 200 | 14 | 10 | 16 | 10 |
| 20% Chol | 200 | 200 | 200 | 200 | 21 | 10 | 12 | 8 |
| 40% Chol | 15 | 197 | 200 | 200 | 12 | 7 | 9 | 9 |
| | | | | | | | | |

| SCR/PLK | 0,33 | 0,5 | 0,67 | 0,82 | 1+ | 1,22+ | 1,5+ | 2+ |
|---|---|---|---|---|---|---|---|---|
| 0% Chol | no effect | no effect | no effect | no effect | 2,40 | 1,59 | 2,65 | 1,63 |
| 20% Chol | 22,42 | no effect | no effect | no effect | 3,45 | 1,65 | 2,07 | 1,29 |
| 40% Chol | 2,48 | 1,16 | no effect | no effect | 1,93 | 1,09 | 1,48 | 1,55 |

## Claims

1. A lipid assembly comprising a cationic lipid comprising a guanido moiety and an anionic lipid comprising a carboxyl group, further **characterized in that** the ratio between the guanido moiety of the cationic lipid and the carboxyl group of the anionic lipid is less than or equal to 1.5.

2. A lipid assembly as in claim 1, further **characterized in that** the assembly comprises about equal molar amounts of the guanido moiety and the carboxyl group.

3. A lipid assembly as in claim 1, further **characterized in that** said assembly comprises a molar excess of the carboxyl group over the guanido moity.

4. A lipid assembly as in any of the claims 1 to 3, further **characterized in that** said assembly is a liposome.

5. A liposome as in claim 4, further comprising a neutral or zwitterionic lipid selected from cholesterol, phosphatidylcholine, phosphatidylethanolamine, sphingomyeline or mixtures thereof.

6. A liposome as in claim 4 or claim 5, further comprising PEG moieties grafted to their lipid phase.

7. A liposome as in any of the preceding claims, further comprising an active pharmaceutical ingredient.

8. A liposome as in claim 7, wherein said pharmaceutical ingredient is an oligonucleotide.

9. A liposome as in claim 8, wherein said oligonucleotide is a decoy oligonucleotide, and antisense oligonucleotide, a siRNA, an agent influencing transcription, a ribozyme, DNAzyme or an aptamer.

10. A liposome as in claim 9, wherein said oligonucleotides comprise modified nucleosides such as DNA, RNA, LNA, PNA, 2'OMe RNA, 2' MOE RNA, 2'F RNA in their phosphodiester or phosphothioate forms.

11. A liposome as claimed in any of claims 1 to 10 for use in the *in vivo* transfection of cells.

12. Use of the liposome as claimed in any of claims 1 to 10 for the *in vitro* or *ex vivo* transfection of cells.

## Patentansprüche

1. Lipid-Zusammensetzung, umfassend ein einen Guanidorest enthaltendes kationisches Lipid und ein eine Carboxylgruppe umfassendes anionisches Lipid, ferner **dadurch gekennzeichnet, dass** das Verhältnis zwischen dem Guanidorest des kationischen Lipids und der Carboxylgruppe des anionischen Lipids kleiner als oder gleich 1,5 ist.

2. Lipid-Zusammensetzung nach Anspruch 1, ferner **dadurch gekennzeichnet, dass** die Zusammensetzung etwa gleiche molare Mengen des Guanidorests und der Carboxylgruppe umfasst.

3. Lipid-Zusammensetzung nach Anspruch 1, ferner **dadurch gekennzeichnet, dass** die Zusammensetzung einen molaren Überschuss an der Carboxylgruppe gegenüber dem Guanidorest umfasst.

4. Lipid-Zusammensetzung nach einem der Ansprüche 1 bis 3, ferner **dadurch gekennzeichnet, dass** die Zusammensetzung ein Liposom ist.

5. Liposom nach Anspruch 4, ferner umfassend ein neutrales oder zwitterionisches Lipid, ausgewählt aus Cholesterol, Phosphatidylcholin, Phosphatidylethanolamin, Sphingomyelin oder Mischungen daraus.

6. Liposom nach Anspruch 4 oder Anspruch 5, ferner umfassend PEG-Reste, die an ihre Lipidphase aufgepfropft sind.

7. Liposom nach einem der vorherigen Ansprüche, ferner umfassend einen aktiven pharmazeutischen Inhaltsstoff.

8. Liposom nach Anspruch 7, bei dem der pharmazeutische Inhaltsstoff ein Oligonukleotid ist.

9. Liposom nach Anspruch 8, bei dem das Oligonukleotid ein Decoy-Nukleotid und antisense-Nukleotid, eine siRNA, ein die Transkription beeinflussender Wirkstoff, ein Ribozym, ein DNAzym oder ein Aptamer ist.

10. Liposom nach Anspruch 9, bei dem die Oligonukleotide modifizierte Nukleoside umfassen, wie etwa DNA, RNA, LNA, PNA, 2'OMe RNA, 2' MOE RNA, 2'F RNA in ihren Phosphodiester- oder Phosphothioatformen.

11. Liposom nach einem der Ansprüche 1 bis 10 zur Verwendung in *der in vivo-*Transfektion von Zellen.

12. Verwendung des Liposoms nach einem der Ansprüche 1 bis 10 für *die in vitro-* oder ex vivo-Transfektion von Zellen.

## Revendications

1. Assemblage lipidique comprenant un lipide cationique comprenant un fragment guanido et un lipide anionique comprenant un groupe carboxyle, qui est en outre **caractérisé en ce que** le rapport entre le fragment guanido du lipide cationique et le groupe carboxyle du lipide anionique est inférieur ou égal à 1,5.

2. Assemblage lipidique selon la revendication 1, qui est en outre **caractérisé en ce que** l'assemblage comprend des quantités environ équimolaires du fragment guanido et du groupe carboxyle.

3. Assemblage lipidique selon la revendication 1, qui est en outre **caractérisé en ce que** ledit assemblage comprend un excès molaire du groupe carboxyle par rapport au fragment guanido.

4. Assemblage lipidique selon l'une quelconque des revendications 1 à 3, qui est en outre **caractérisé en ce que** ledit assemblage est un liposome.

5. Liposome selon la revendication 4, comprenant en outre un lipide neutre ou zwittérionique sélectionné parmi le cholestérol, la phosphatidylcholine, la phosphatidyléthanolamine, la sphingomyéline ou des mélanges de ceux-ci.

6. Liposome selon la revendication 4 ou la revendication 5, comprenant en outre des fragments de PEG greffés à leur phase lipidique.

7. Liposome selon l'une quelconque des revendications précédentes, comprenant en outre un ingrédient pharmaceutique actif.

8. Liposome selon la revendication 7, dans lequel ledit ingrédient pharmaceutique est un oligonucléotide.

9. Liposome selon la revendication 8, dans lequel ledit oligonucléotide est un oligonucléotide leurre, et un oligonucléotide antisens, un ARNsi, un agent influençant la transcription, un ribozyme, une enzyme ADN (ADNzyme) ou un aptamère.

10. Liposome selon la revendication 9, dans lequel lesdits oligonucléotides comprennent des nucléosides modifiés comme de l'ADN, ARN, LNA, PNA, ARN 2'OMe, ARN 2' MOE, ARN 2'F dans leurs formes phosphodiester ou phosphorothioate.

11. Liposome selon l'une quelconque des revendications 1 à 10, destiné à être utilisé dans la transfection de cellules *in vivo.*

12. Utilisation du liposome selon l'une quelconque des revendications 1 à 10, pour la transfection de cellules *in vitro* ou *ex vivo.*
